# EUROPÄISCHE PATENTSCHRIFT

(11) **EP 1 124 484 B1**
(45) Veröffentlichungstag und Bekanntmachung des Hinweises auf die Patenterteilung: **15.09.2004**
(21) Anmeldenummer: 98949857.1
(22) Anmeldetag: 04.11.1998
(51) Int. Cl.: A61B 5/103, A61B 5/107

(54) **MESSANORDNUNG ZUM ERFASSEN EINER OBERFLÄCHENLINIE EINES KÖRPERS**
MEASURING SYSTEM FOR DETERMINING THE SURFACE LINE OF A BODY
SYSTEME DE MESURE POUR DETECTER LA LIGNE SUPERFICIELLE D'UN CORPS

(43) Veröffentlichungstag der Anmeldung: 22.08.2001
(73) Patentinhaber: Idiag, 8604 Volketswil (CH)
(72) Erfinder: FASANELLA, Piero, CH-8306 Brüttisellen (CH)
(74) Vertreter: Bruderer, Werner
(86) Internationale Anmeldenummer: PCT/CH1998/000467
(87) Internationale Veröffentlichungsnummer: WO 2000/025674

(56) Entgegenhaltungen:
- EP-A- 0 487 339
- DE-C- 4 090 228
- FR-A- 2 529 776
- GB-A- 2 045 938

## Beschreibung

Die Erfindung betrifft eine Messanordnung zum Erfassen der Form und Länge einer Oberflächenlinie eines Körpers mit einem frei beweglichen Messgerät, welches mit einer Messeinrichtung zur Messung der Länge eines Verschiebeweges des Messgerätes entlang der Oberflächenlinie und einer Messeinrichtung zur Bestimmung von Winkelveränderungen einer Messachse des Messgerätes zu einer vorgegebenen Referenzachse ausgestattet ist, mit einer Datenübertragungseinrichtung zu einem Computer und einem Computer, welcher die Weg- und Winkelmesswerte der beiden Messgeräte verarbeitet und eine Darstellung der Oberflächenlinie erzeugt.

Derartige Messanordnungen finden insbesondere zur Erfassung und Abtastung der Form und Länge von Körperkonturen und von Bewegungsbereichen bei gelenkigen Körpern, insbesondere am menschlichen Körper Verwendung. Von besonderem Interesse sind dabei die Erfassung der Form und Länge des Verlaufs der Wirbelsäule und Messungen zur Überprüfung deren Beweglichkeit, aber auch die Messung des Bewegungsablaufes an anderen Gelenken, wie z.B. Hüft- oder Kniegelenken. Eine Messanordnung dieser Art ist beispielsweise aus DE 40 90 228 C1 bekannt, in welcher auch verschiedene Anwendungsmöglichkeiten im Bereiche von Messungen an der Wirbelsäule beschrieben sind. Bei dieser bekannten Anordnung ist ein frei bewegliches Messgerät vorhanden, welches mit einem Computer zur Auswertung und Darstellung der Daten verbunden ist. Im beweglichen Messgerät ist eine Messeinrichtung zur Messung der Länge des Verschiebeweges des Messgerätes vorhanden, und zwar ein elektrischer Wegmessaufnehmer. Diese Wegmesseinrichtung verfügt über Laufrollen oder Walzen, welche bei der Verschiebung des Messgerätes auf der auszumessenden Oberfläche, bzw. Linie abrollen und über an sich bekannte Mittel zur Umformung dieser Abrollbewegung in elektrische Signale, z.B über einen inkrementalen Weggeber. Im weiteren verfügt das Messgerät auch über eine Winkelmesseinrichtung in der Form einer Lotpendeleinrichtung. Diese Lotpendeleinrichtung ist so ausgebildet, dass sie in zwei um 90° verschwenkten Positionen eingesetzt werden kann. Dies ermöglicht, in einem ersten Messvorgang durch Abfahren der Oberflächenlinie mit dem beweglichen Messgerät Krümmungen in einer Richtung festzustellen und durch ein Wiederholen des Abfahrvorganges und Umstellen der Lotpendeleinrichtung um 90° Krümmungen der Oberflächenlinie in einer rechtwinklig dazu stehenden Ebene festzustellen. Zur Feststellung der Krümmung und Form der Oberflächenlinie werden zu bestimmten Punkten, bzw. Streckenabschnitten dieser Oberflächenlinie die entsprechenden Winkelabweichungen über das Lotpendel ermittelt und daraus die Krümmung der Oberflächenlinie festgestellt. Die für die Winkelmessung zum Einsatz gelangenden Lotpendel stellen relativ empfindliche und auch entsprechend teure Messgeräte dar, und sie haben in den handelsüblichen Ausführungen auch nur einen beschränkten Winkelmessbereich. Wenn bei Messungen am menschlichen Körper, z.B. bei Patienten mit Rückenschwierigkeiten, Messungen am stehenden und auch am liegenden Körper durchgeführt werden müssen, verlangen diese unterschiedlichen Messungen eine Umstellung des Messgerätes, bzw. der Lotpendeleinrichtung, auf die jeweilige Lage des Patienten. Als Folge muss auch die Messelektronik jeweils neu initiiert und der Ausgangspunkt der Messung entsprechend gestartet werden. Dies ist zeitaufwendig und kann auch zu Abweichungen der Messergebnisse und Fehlern führen, da zwischenzeitliche Bewegungen des Patienten nicht auszuschliessen sind.

Eine weitere Messanordnung zur Erfassung der Rückenkontur eines Menschen ist aus DE 44 02 562 A1 bekannt geworden. Bei dieser Anordnung wird im beweglichen Messgerät ebenfalls ein Lotpendel für die Winkelmessung eingesetzt. Dieses Lotpendel hat wohl einen vergrösserten Winkelmessbereich, weist jedoch weiterhin den Nachteil auf, dass Lotpendel empfindliche Messgeräte mit einem komplizierten inneren Aufbau sind. Sie sind deshalb entsprechend teuer und erfordern auch eine sorgfältige Handhabung und einen korrekten Einsatz. Bei den Messungen muss das Lotpendel möglichst in einer Vertikalebene stehen, da sonst durch die Dämpfung die Messresultate verfälscht werden können. Bei zu grosser Abweichung aus der Vertikalebene können Messungen sogar unmöglich werden.

In der Praxis treten deshalb immer wieder Schwierigkeiten auf, da das Messobjekt, an welchem die Form und Länge einer Oberflächenlinie erfasst werden soll, in eine Position gebracht werden muss, welche dem zulässigen Messbereich der Messanordnung entspricht. insbesondere bei Messungen am menschlichen Körper und dort, wo Messungen oder Folgen von Messreihen rasch ausgeführt werden sollten, erschwert dies die Messabläufe. Die bekannten Messanordnungen bedingen deshalb eine entsprechende Schulung und Übung in der Anwendung. Auch bei entsprechend trainierten Anwendem ist der Zeitaufwand zur Durchführung von Messungen teilweise noch erheblich, wobei insbesondere die Umstellungen der Winkelmesseinrichtung und die jeweilige Initialisierung zeitaufwendig sind.

Aus GB 2 045 938 ist eine weitere Einrichtung zur Messung der Dimensionen von Objekten bekannt. Dabei handelt es sich um eine Messpistole mit einer abstehenden Messspitze. Mit Abstand zur Messspitze sind in der Messpistole sechs Beschleunigungssensoren angeordnet, um die Beschleunigungswerte des vorderen Endes der Messspitze in den sechs Freiheitsgrade zu ermitteln. Die Einrichtung ist dazu bestimmt, Positionspunkte des Objektes, welche einen Abstand zueinander aufweisen, auszumessen, d.h. sie ermöglicht Punkt zu Punkt Messungen.

Es ist Aufgabe der vorliegenden Erfindung, eine Messanordnung, bzw. ein Messgerät zu schaffen, mit welchem die Erfassung von Form und Länge von Oberflächenlinien eines Körpers in einer Ebene über einen Winkelbereich von 0 bis 360° möglich ist, bei Lageveränderungen des Messgerätes oder bei wechselnden Messvorgängen keine Initialisierung und Eichung des Messgerätes notwendig ist, die Messeinrichtung zur Bestimmung der Winkelveränderungen im Raum freier beweglich und einfacher aufgebaut sein soll und es soll der Verlauf der Oberflächenlinie im gleichen Messvorgang in Bezug auf zwei rechtwinklig zueinander stehenden Messflächen oder als Raumvektor feststellbar sein.

Diese Aufgabe wird durch die im kennzeichnenden Teil des Patentanspruches 1 definierten Merkmale gelöst. Vorteilhafte Weiterbildungen der Erfindung ergeben sich nach den Merkmalen der abhängigen Patentansprüche.

Bei der erfindungsgemässen Messanordnung ist das bewegliche Messgerät mit einer an sich bekannten Messeinrichtung zur Messung der Länge eines Verschiebeweges entlang einer Oberflächenlinie, wie sie im Stand der Technik beschrieben ist, ausgestattet. In Kombination mit dieser an sich bekannten Längenmesseinrichtung ist die Messeinrichtung, zur Bestimmung von Winkelveränderungen einer Messachse des Messgerätes, mit zwei Beschleunigungssensoren ausgebildet. Die Verwendung von Beschleunigungssensoren zur Bestimmung von Winkelveränderungen der Messeinrichtung erbringt den Vorteil, dass Sensoren eingesetzt werden können, welche eine genau definierte Messachse aufweisen und zudem keine beweglichen Teile vorhanden sind, welche um eine Achse schwenkbar gelagert sein müssen oder mit Dämpfungselementen ausgestattet sind. Dadurch wird der Aufbau der Messeinrichtung zur Bestimmung von Winkelveränderungen wesentlich vereinfacht, und die Störungsanfälligkeit erheblich reduziert. Bei den zur Verwendung vorgeschlagenen Beschleunigungssensoren handelt es sich um Sensoren, welche normalerweise dazu eingesetzt werden, Beschleunigungen und Verzögerungen von bewegten Objekten in Richtung ihrer Messachse zu ermitteln. Derartige, an sich bekannte Sensoren, weisen aber auch die Eigenschaft auf, dass sie auch im stationären Zustand, d.h. ohne Bewegungskomponente in Richtung ihrer Messachse bei Winkelveränderungen der Messachse Messsignale abgeben. Dieser Effekt ist auf die normale Gravitationskraft, bzw. Erdbeschleunigung zurückzuführen, welche immer auf den Sensor wirkt. Wenn ein Beschleunigungssensor so ausgerichtet ist, dass die Messachse parallel zur Gravitationsachse steht, wirkt die volle Erdbeschleunigung auf das Messelement des Beschleunigungssensors. Liegt die Messachse des Beschleunigungssensors genau rechtwinklig zur Gravitationsachse, so wird das Messelement des Beschleunigungssensors nicht ausgelenkt, und es wirkt auch keine Komponente der Erdbeschleunigung in Richtung der Messachse. Je nach Winkelstellung zwischen 0 und 90° erzeugt der Beschleunigungssensor unterschiedliche Messsignale, aus welchen die Winkelstellung der Messachse des Beschleunigungssensors zur - Gravitationsachse abgeleitet werden kann. Die bekannten Beschleunigungssensoren bestehen aus einem Sensor und einer integrierten Schattung, welche normalerweise als geschlossene Einheit mit einem Stromanschluss und einem Signalausgang ausgebildet ist. Die Abhängigkeit der vom Beschleunigungssensor abgegebenen Signale ist jedoch nicht linear vom Winkel abhängig, sondern die Empfindlichkeit ist im Bereiche um 90° zur Gravitationsachse am grössten und ist im Bereiche, wo die Messachse in eine Parallellage zur Gravitationsachse bewegt wird, gering.

Es ergibt sich nun ein weiterer Vorteil, wenn in einer Messebene zwei Beschleunigungssensoren angeordnet werden, deren Messachsen in dieser gemeinsamen Ebene liegen und rechtwinklig zueinander stehen. Werden die Ausgangssignale dieser beiden Beschleunigungssensoren miteinander verknüpft, so ergibt sich eine eindeutige Zuordnung zu einem bestimmten Winkel gegenüber der Gravitationsachse und gleichzeitig eine hohe Genauigkeit, da immer einer der beiden Sensoren im Bereiche der hohen Empfindlichkeit wirksam ist. Da die beiden Beschleunigungssensoren nicht von einer Drehachse abhängig sind, sondern ihre Messachse grundsätzlich beliebig im Raum angeordnet sein kann, ergibt sich der Vorteil, dass die Ebene, in welcher die beiden Messachsen der Beschleunigungssensoren angeordnet sind, im Messgerät so ausgerichtet werden kann, dass auch die Messachse der Messeinrichtung für den Verschiebeweg in dieser gemeinsamen Ebene liegt. Die Ausgangssignale der Beschleunigungssensoren werden einem Messwertaufnehmer zugeführt, und dieser ist über eine Schnittstelle und eine Datenleitung mit einem Computer, vorteilhafterweise mit einem Personal Computer, verbunden. Diese Datenleitung kann durch ein Kabel gebildet sein, wobei sich jedoch eine besonders vorteilhafte Lösung ergibt, wenn die Daten des Messwertaufnehmers drahtlos an den Computer übermittelt werden. Dadurch wird die freie Beweglichkeit des Messgerätes erhöht, und es ist für das Bedienungspersonal leichter handhabbar.

Durch die Verwendung eines dritten Beschleunigungssensors kann in einfacher und vorteilhafter Weise eine zweite Messeinrichtung zur Bestimmung von Winkelveränderungen gebildet werden, indem dieser dritte Beschleunigungssensor mit einem der beiden Sensoren der ersten Messeinrichtung zur Bestimmung von Winkelveränderungen zu einer zweiten Messeinrichtung zusammengefasst wird.

Die Messachse dieses dritten Beschleunigungssensors wird dabei in einer Ebene angeordnet, welche rechtwinklig zur Ebene steht, welche durch die Messachsen der beiden ersten Beschleunigungssensoren gebildet ist. Dadurch, dass je zwei Sensoren zu einer ersten und zu einer zweiten Messeinrichtung zusammengefasst sind, lassen sich Winkelveränderungen in zwei senkrecht zueinander stehenden Ebenen feststellen. Der Messbereich erstreckt sich dabei von 0 bis 360° in jeder der beiden Ebenen, da immer einer der Sensoren im empfindlichen Messbereich liegt. Da die Charakteristik der Signalkurve in Abhängigkeit vom Winkel der Messachse zur Gravitationsachse genau bekannt ist, lassen sich die Winkel sehr genau und über den ganzen Bereich feststellen. Die erfindungsgemässe Messanordnung bietet zusätzlich den Vorteil, dass unterschiedliche Fabrikate von Beschleunigungssensoren eingesetzt werden können, da deren Signal-, bzw. Messcharakteristik, von vornherein bekannt ist. Durch entsprechende Auswertung der Messsignale im Computer mit einer geeigneten Software können beliebige Winkel im Raum sowohl zur X-, wie Y-, wie auch Z-Achse festgestellt werden. Bei Bedarf können diese Messsignale auch in Vektoren umgeformt werden. In Verbindung mit den Messwerten aus der Wegmessung werden die Messwerte der Winkelmessung zur Darstellung des Verlaufs von Oberflächenlinien eines Körpers verwendet.

Zur Erfassung der Form und Länge einer Oberflächenlinie eines Körpers, z.B. der Form der Wirbelsäule eines Menschen, wird das Messgerät entlang der Wirbelsäule, bzw. der Oberflächenlinie, verschoben. Dabei nimmt die Messeinrichtung zur Messung der Länge des Verschiebeweges die entsprechenden Verschiebebewegungen auf, und über einen Messwertaufnehmer werden die entsprechenden Messwerte als Daten an den Computer übermittelt. In vorbestimmten Wegund/oder Zeitabschnitten werden dazu über die Messeinrichtungen zur Bestimmung von Winkelveränderungen die Neigungswinkel der Oberflächenlinie festgestellt. Aus den zu einem bestimmten Messpunkt gehörenden Daten wird der Verlauf der Oberflächenlinie im Bereiche dieses Messpunktes errechnet und dann aus der Vielzahl von Messpunkten der Verlauf der Gesamtkurve, bzw. der gesamten Oberflächen linie, ermittelt. Diese kann dann auf einem an sich bekannten Ausgabegerät, wie einem Drucker oder einem Bildschirm, dargestellt, bzw. ausgegeben und einem Betrachter zugänglich gemacht werden. Während eines Bewegungsvorganges des Messgerätes können Zwischenzustände der Bewegungskurve und Endzustände festgestellt und dargestellt werden. Die erfindungsgemässe Messanordnung benötigt keine Eichung in der Ausgangsposition, da aufgrund der Messwerte der Sensoren immer genau festgestellt werden kann, welche Position die Messachsen des Messgerätes in Bezug auf die Gravitationsachse einnehmen. Dies erleichtert den Ablauf von Messvorgängen, z.B. an Patienten mit Rücken- oder Gelenkbeschwerden erheblich, da diese nicht gezwungen sind, eine bestimmte Messposition einzunehmen. Für Standardmessungen ist es sicher zweckmässig, mindestens von einer oder mehreren näherungsweisen Normalpositionen auszugehen. Dies erleichtert den Vergleich von Messvorgängen untereinander und auch die Auswertung der angezeigten Resultate. Bei genügender Erfahrung des Bedienungspersonals und dem Einsatz der geeigneten Software ermöglicht die erfindungsgemässe Messanordnung aber auch Messungen in beliebigen Positionen, d.h. der Anwendungsbereich dieser Messanordnung wird erheblich erweitert. Trotz dieser Erweiterung des Anwendungsbereiches ist das Messgerät leicht handhabbar und nicht störungsanfällig.

Die Anordnung eines Eingabegerätes mit mindestens einer Steuertaste am beweglichen Messgerät erbringt den weiteren Vorteil, dass die Bedienbarkeit verbessert wird, indem Steuerfunktionen für die Datenverarbeitung im PC über diese Steuertasten angesteuert werden können und zum Beispiel während den Messvorgängen die eigentliche Bedienungstastatur des Computers durch diese Steuertasten ersetzt wird. Die daraus resultierende Erleichterung der Arbeit ist erheblich und ermöglicht ein schnelleres und genaueres Arbeiten. Eine weitere Verbesserung ergibt sich durch die Anordnung einer Anzeigevorrichtung am Messgerät. Diese Anzeigevorrichtung kann durch eine Leuchtdiode (LED) oder durch eine andere, an sich bekannte Vorrichtung, wie eine Flüssigkristallanzeige (LCD) gebildet sein. Eine Leuchtdiode könnte bestimmte Betriebszustände optisch anzeigen. Bei Einsatz einer Flüssigkristallanzeige ergibt sich die erweiterte Möglichkeit zur Anzeige in Symbolen, Ziffern oder Texten. Diese Anzeigevorrichtung ermöglicht den Bedienungspersonen die ganze Aufmerksamkeit auf den Messvorgang und das Messgerät zu richten, da alle Betriebsmeldungen am Messgerät angezeigt werden können. Auch dies führt zu einer zusätzlichen Erleichterung und Beschleunigung des Messvorganges.

Nachfolgend wird die Erfindung mit Hilfe von Zeichnungen, welche Ausführungsbeispiele darstellen, näher erläutert. Es zeigen:
- Fig. 1: eine perspektivische Ansicht eines Messgerätes der erfindungsgemässen Messanordnung,
- Fig. 2: die erfindungsgemässe Messanordnung in schematischer Darstellung,
- Fig. 3: ein Diagramm mit den Ausgangssignalen eines Sensorpaares in Abhängigkeit des Messwinkels, und
- Fig. 4: eine schematische Darstellung einer Messposition, rechtwinklig zu der in Fig. 2 gezeigten Messebene.

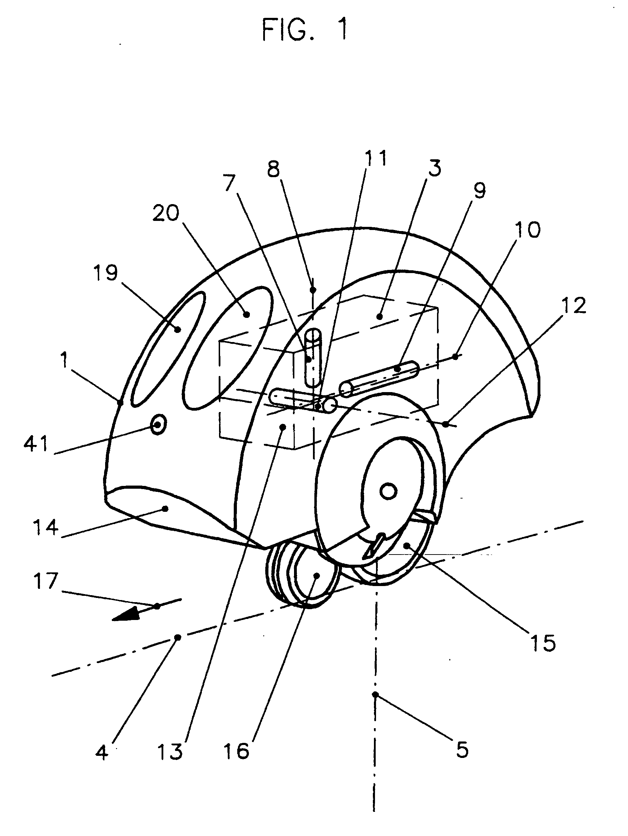

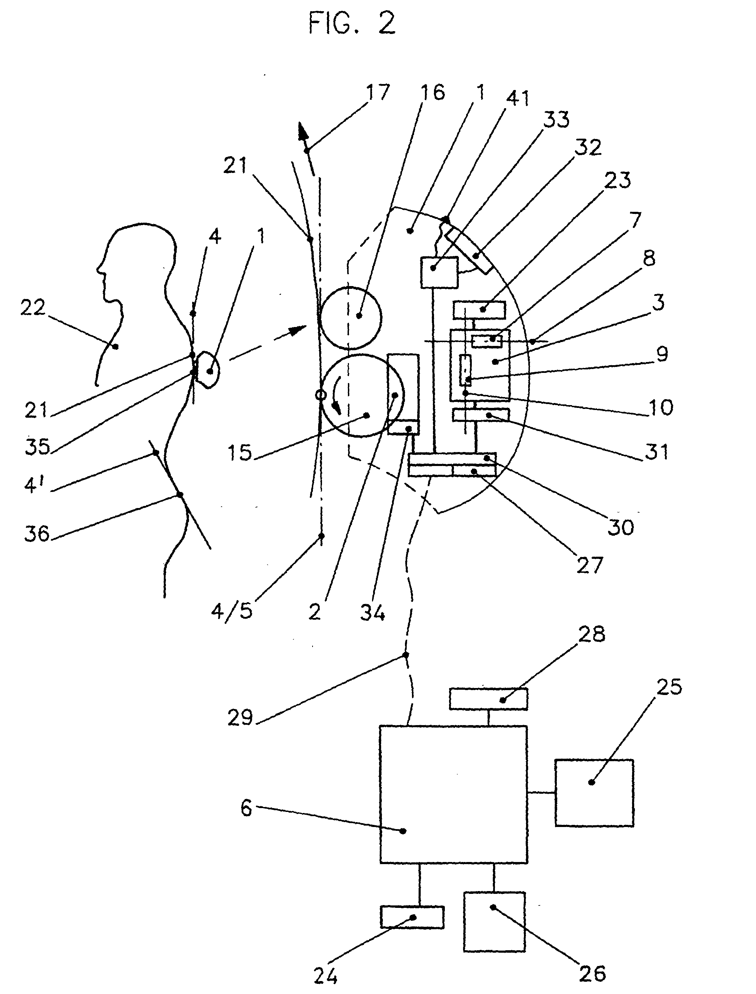

Fig. 1 zeigt ein frei bewegliches erfindungsgemässes Messgerät 1. Dieses Messgerät 1 umfasst ein ergonomisch geformtes Gehäuse 14, welches in einfacher und komfortabler Weise in einer Hand gehalten werden kann. Im Gehäuse 14 ist ein Messrad 15 und ein Führungsrad 16 gelagert. Das Führungsrad 16 und das Messrad 15 sind auf eine Führungsachse 4 ausgerichtet und sind Teile einer Messeinrichtung 2 (siehe Fig. 2) zur Messung der Länge von Verschiebewegen des Messgerätes 1 in Richtung des Pfeiles 17 entlang der Messachse 4. Im weiteren verfügt das Messgerät 1 über zwei Tasten 19, 20, welche Teil eines in Fig. 2 dargestellten Eingabegerätes 32 sind. Im Gehäuse 14 des Messgerätes 1 sind im weiteren eine erste Messeinrichtung 3 und eine zweite Messeinrichtung 13 zur Messung von Winkelabweichungen des Messgerätes 1, bzw. der Messachse 4 gegenüber einer Referenzachse 5 angeordnet. Die beiden Messeinrichtungen 3 und 13 sind dabei nur schematisch angedeutet. Die Referenzachse 5 wird durch die Achse der Richtung der Erdbeschleunigung, d.h. durch die Gravitationsachse gebildet und ist deshalb in jedem Fall definiert und vorgegeben. Die erste Messeinrichtung 3 zur Bestimmung von Winkelveränderungen der Messachse 4 umfasst einen ersten Beschleunigungssensor 7 mit einer Messachse 8 und einen zweiten Beschleunigungssensor 9 mit einer Messachse 10. Die beiden Messachsen 8 und 10 der beiden Beschleunigungssensoren 7 und 9 liegen dabei in einer gemeinsamen Ebene und stehen rechtwinklig zueinander. Die Messachse 10 des Beschleunigungssensors 9 liegt in der in Fig. 1 dargestellten Position des Messgerätes 1 rechtwinklig zur Referenzachse 5 und die Messachse 8 des Beschleunigungssensors 7 verläuft parallel dazu. Die Messeinrichtung 3, welche die beiden Beschleunigungssensoren 7 und 9 umfasst, ist dabei im Gehäuse 14 des Messgerätes 1 so eingebaut, dass die von den beiden Achsen 8 und 10 definierte Messebene in der gleichen Ebene wie die Messachse 4 der Messeinrichtung 2 für die Längenmessung liegt Mit dieser ersten Messeinrichtung 3 zur Bestimmung von Winkelveränderungen werden Winkelveränderungen des Messgerätes 1 gegenüber der Referenzachse 5 festgestellt, welche bei Drehungen des Messgerätes 1 in der durch die Messachse 4 und die Referenzachse 5 gebildeten Ebene auftreten. Mit dem zweiten Beschleunigungssensor 9 und einem dritten Beschleunigungssensor 11 ist eine zweite Messeinrichtung 13 für Winkeiveränderungen ausgebildet, wobei die Messachsen 10 und 12 dieser beiden Beschleunigungssensoren 9 und 11 ebenfalls rechtwinklig zueinander stehen und eine Messebene definieren, welche rechtwinklig zu derjenigen steht, in welcher die beiden Messachsen 8 und 10 der Beschleunigungssensoren 7 und 9 der ersten Messeinrichtung 3 für Winkelveränderungen liegen. Diese zweite Messeinrichtung 13 für Winkelveränderungen kommt insbesondere dann zum Einsatz, wenn das Messgerät 1 in einer etwa vertikalen Position eingesetzt wird, d.h. wenn die Messachse 4 für die Längenmessung etwa in Richtung der Referenzachse 5 verläuft. Bei den Beschleunigungssensoren 7, 9 und 11 handelt es sich um handelsübliche elektronische Bauelemente, welche in einen elektronischen Schaltkreis integriert sind. Jeder der drei Beschleunigungssensoren 7, 9 und 11 ist für sich allein funktionsfähig und ist dazu mit einer Energieversorgung, bzw. einem Stromanschluss und einem Signalausgang versehen. Die Zusammenfassung von je zwei Beschleunigungssensoren 7, 9 bzw. 9, 11 zu einer Messeinrichtung 3, bzw. 13 für Winkelveränderungen, ermöglicht genaue Winkelmessungen von 0 bis 360° in den durch die Messachsen 8, 10, bzw. 10, 12 definierten Ebenen. Die erfindungsgemässe Anordnung von drei Beschleunigungssensoren 7, 9, 11 ermöglicht deshalb die gleichzeitige Messung von Winkelveränderungen in zwei senkrecht zueinander stehenden Vertikalebenen, bzw. von zwei Winkelpositionen des Messgerätes 1 in Bezug auf die Referenzachse 5. Diese Winkelwerte können bestimmten Positionen einer Oberflächenlinie 21 (gem. Fig. 2), welche durch die Messeinrichtung 2 zur Messung der Länge der Oberflächenlinie 21 ermittelt werden, zugeordnet werden. Aus diesen Daten kann wie nachfolgend beschrieben, die Form einer Kurve im Raum, z.B. der Oberflächenlinie 21 ermittelt werden. Das Messgerät 1 ist zusätzlich mit einer Leuchtdiode (LED) 41 ausgestattet, welche eine Anzeigevorrichtung, bzw. ein Ausgabegerät für optische Informationen bildet. Durch den Zustand hell oder dunkel und/oder durch unterschiedliche Farbanzeigen, z.B. rot/grün, können bestimmte Zustände des Messvorganges angezeigt werden. Bei Bedarf kann die LED auch durch eine Flüssigkristallanzeige ersetzt werden. Dies ermöglicht die Anzeige, bzw. Ausgabe von umfangreicheren Informationen, z.B. Texten oder Symbolen.

In Fig. 2 ist die Messanordnung zum Erfassen der Form und Länge einer Oberflächenlinie 21 eines Körpers 22 schematisch dargestellt. Im dargestellten Beispiel wird das Messgerät 1 dazu verwendet, an einem menschlichen Körper 22 die Länge und Form der Wirbelsäule, d.h. deren Oberflächenlinie 21 in einer Sagittalebene zu ermitteln. Dabei ist der Körper 22 in stehender Position dargestellt, dieser kann sich aber auch in gebückter oder liegender Position befinden. Im rechten Teil der Fig. 2 ist das Messgerät 1 vergrössert schematisch dargestellt. Das Messgerät 1 ist mit einem Computer 6 verbunden, welcher mit Hilfe einer geeigneten Software die vom Messgerät 1 ermittelten Messdaten verarbeitet und eine Darstellung der Oberflächenlinie 21 der Wirbelsäule des Körpers 22 erzeugt. Der Computer 6 ist in an sich bekannter Weise mit einem Eingabegerät in der Form einer Tastatur 24, einem Bildschirm 25, einem Drucker 26 und allfälligen weiteren Hardware-Elementen verbunden. In dem in Fig. 2 dargestellten Beispiel ist zur Übertragung der Daten zwischen dem Messgerät 1 und dem Computer 6 eine Einrichtung zur drahtlosen Übertragung von Daten vorgesehen. Dazu befindet sich am Messgerät 1 und am Computer 6 je eine Sende-/Empfangseinheit 27, bzw. 28, welche in bekannter Weise zur Datenübertragung, z.B. mittels Funk oder Infrarotsignalen, geeignet sind. Wie durch die gestrichelte Linie 29 angedeutet ist, kann aber auch ein Kabel als Datenleitung eingesetzt werden. Dies reduziert jedoch die freie Beweglichkeit des Messgerätes 1. Das Messgerät 1 ist mit einer Schnittstelle 30 ausgestattet, mit welcher über Datenleitungen alle Messeinrichtungen 2, 3, und 13 des Messgerätes 1 verbunden sind. Zur Messung des Verschiebeweges des Messgerätes 1 in Richtung des Pfeiles 17 entlang der Oberflächenlinie 21 ist das Messgerät 1 mit der Messeinrichtung 2 zur Längenmessung ausgestattet. Bei Verschiebungen des Messgerätes 1 in Richtung des Pfeiles 17 rollt das Messrad 15 auf der Oberfläche des Körpers 22 ab. Die daraus resultierende Drehbewegung des Messrades 15 um eine Achse wird inkremental erfasst, und die entsprechenden Daten werden über einen Messwertaufnehmer 34 der Schnittstelle 30 zugeführt. Im weiteren ist ein Eingabegerät 32 mit mindestens einer Bedienungstaste, vorzugsweise zwei Bedienungstasten 19, 20, vorhanden, welches mit einem Mikroprozessor 33 verknüpft ist. Im dargestellten Beispiel ist dieser Mikroprozessor 33 mit einem Datenspeicher sowie einer Ein-/Ausgabeeinheit-ausgestattet, wobei der Datenspeicher die Zwischenspeicherung von Weg- und Winkelmessdaten ermöglicht. Der Zugriff auf diese Daten ist über das Eingabegerät 32 am Messgerät 1 oder über ein Eingabegerät des Computers 6, z.B. über die Tastatur 24 möglich. Der Mikroprozessor 33, bzw. dessen Ein-/Ausgabeeinheit verfügt über ein Schaltelement, welches die Steuerung wahlweise dem Eingabegerät 32 am Messgerät 1 oder dem Eingabegerät, bzw. Tastatur 24 am Computer 6 zuordnet. Das entsprechende Schaltelement kann auch an einem Prozessor im Computer 6 angeordnet sein. Über die Ein-/Ausgabeeinheit des Mikroprozessors 33 wird auch die optische Anzeigevorrichtung 41 gesteuert. Im dargestellten Beispiel handelt es sich um eine Leuchtdiode, welche bestimmte Betriebszustände des Messvorganges durch verschiedene Farben und die Zustände leuchtend oder nicht leuchtend anzeigt. Die Bereitschaft für den Beginn der Messung wird beispielsweise durch grün leuchtend angezeigt. Es kann auch zweckmässig sein, mehrere Leuchtdioden anzuordnen und durch den Mikroprozessor 33 zu verbinden. Eine Energiequelle 23, welche aus einer Batterie oder einem aufladbaren Akkumulator besteht, dient der Speisung der Messeinrichtung 2 für die Längenmessung und der Messeinrichtungen 3 und 13 für die Messung von Winkelveränderungen, sowie allfälliger weiterer elektrischen Bauelemente. Die Messeinrichtungen 3 und 13 zur Ermittlung von Winkelveränderungen sind ebenfalls mit einem Messwertaufnehmer 31 verbunden, welcher seinerseits mit der Schnittstelle 30 verknüpft ist. Die in Fig. 2 prinzipiell dargestellte erste Messeinrichtung 3 zur Bestimmung von Winkelveränderungen, bzw. Winkelpositionen des Messgerätes 1 umfasst die zwei Beschleunigungssensoren 7 und 9.

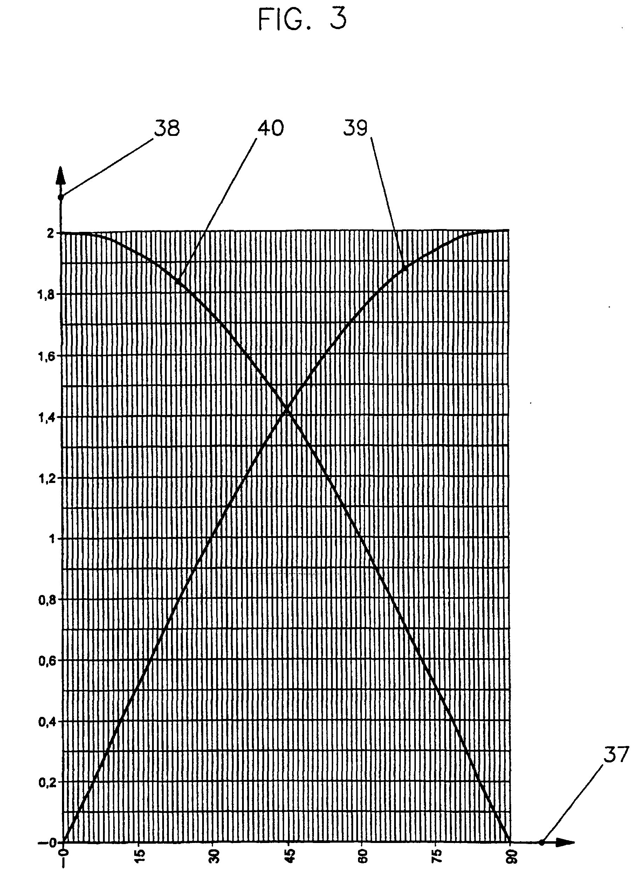

Die in diesem Beispiel eingesetzten Beschleunigungssensoren 7, 9, 11 sind Sensoren an sich bekannter Art und werden üblicherweise dazu eingesetzt, Beschleunigen oder Verzögerungen in Richtung ihrer Messachsen 8, 10, 12 festzustellen. Beim erfindungsgemässen Messgerät 1 wird die Eigenschaft derartiger Beschleunigungssensoren 7, 9, 11 genutzt, dass diese auch im stationären Zustand, d.h. ohne Bewegungskomponente in Richtung ihrer Messachse 8, 10, 12 bei Veränderungen der Lage der Messachse 8, 10, 12 im Verhältnis zur Achse 5 der Erdbeschleunigung Messsignale erzeugen. Die Beschleunigungs-, bzw. Verzögerungskräfte, welche durch die Bewegung des Messgerätes 1 entlang der Oberflächenlinie 21 auf die Sensoren wirken, können dabei vernachlässigt werden, da sie bei den hier auftretenden Bewegungsgeschwindigkeiten keine Veränderung der Signale bewirken. Die Beschleunigungssensoren 7, 9, 11 haben den Vorteil, dass sie je nach Richtung der Veränderung des Winkels gegenüber der Gravitationsachse 5 positive oder negative Signale erzeugen. Damit kann die Richtung der Winkelabweichung bestimmt werden. Bei den normalerweise verfügbaren Beschleunigungssensoren 7, 9, 11 ist die Kennlinie, welche das Verhältnis der Eingangsgrösse zur Ausgangsgrösse angibt, nicht linear. Bei der in der Messeinrichtung 3 getroffenen Anordnung der Sensoren 7 und 9 stehen deren Messachsen 8 bzw. 10 rechtwinklig zueinander. In der in Fig. 2 dargestellten Position definieren die beiden Messachsen 8 und 10 eine Messebene, welche der Bildebene entspricht. Wird das Messgerät 1 beim Verschieben entlang der Oberflächenlinie 21 in dieser Ebene so gekippt, dass sich der Winkel der beiden Messachsen 8 und 10 der Beschleünigirngssensoren 7 und 9 gegenüber der Referenzachse, bzw. Gravitationsachse 5 verändert, so erzeugen die Sensoren 7 und 9 Messignale, welche in Abhängigkeit der Winkelposition die in Fig. 3 dargestellten Kennlinien ergeben.

In dem in Fig. 3 dargestellten Diagramm sind in Richtung der Achse 37 die Winkelveränderungen der Messachsen 8 bzw. 10 im Verhältnis zur Referenzachse 5 aufgetragen und auf der rechtwinklig dazu stehenden Achse 38 die Messsignale z.B. als Spannungswerte. Die Kurve 39 stellt dabei die Kennlinie für den Beschleunigungssensor 7 dar und die Kurve 40 die Kennlinie für den Beschteunigungssensor 9. Aus diesem Kennliniendiagramm ist ersichtlich, dass der Beschleunigungssensor 7 im Bereiche von Winkelveränderungen von 0° bis ca. 60° eine sehr gute Auflösung aufweist. Im Bereiche bis gegen 90° wird jedoch die Auflösung immer schlechter, d.h. das Messergebnis ungenau. Demgegenüber zeigt die Kennlinie des Beschleunigungssensors 9, dass dessen Signale im Bereiche von 0° bis etwa 30° eine schlechte Auflösung, d.h. eine Ungenauigkeit der Messresultate ergeben, und ab ca. 30° bis 90° die Auflösung sehr gut und damit auch die Messgenauigkeit sehr hoch ist. Um trotzdem im gesamten Bereich von 0 bis 360° genaue Messungen durchführen zu können, bilden die beiden Beschteunigungssensoren 7 und 9 ein Paar von Messelementen, und für jede Winkelposition werden die Signale beider Sensoren 7, 9 erfasst. Das daraus resultierende Messsignal-, bzw. Messwertpaar ermöglicht eine genaue Zuordnung zu einem bestimmten Winkel und zwar über den gesamten Bereich von 0 bis 360°. In der in Fig. 2 dargestellten Position des Messgerätes 1, welche dem Messpunkt 35 auf der Oberflächenlinie 21 zugeordnet ist, ergibt sich für den Beschleunigungssensor 7 ein Messwert von 0 und für den Beschleunigungssensor 9 ein Messwert von +2. Damit ist eindeutig festgelegt, dass sich das Messgerät 1 in vertikaler Position befindet und das Führungsrad 16 nach oben gerichtet ist. Wenn das Messgerät 1 um 180° gedreht würde, d.h. das Führungsrad 16 nach unten gerichtet wäre, würde der Beschleunigungssensor 7 weiterhin einen Messwert 0 abgeben, jedoch der Beschleunigungssensor 9 einen Messwert von -2. Die Verarbeitung dieser Messwertdaten erfolgt bei der erfindungsgemässen Messanordnung im Computer 6 mit Hilfe einer entsprechenden Software. Die Verarbeitung kann aber teilweise auch im Mikroprozessor 33 erfolgen, und es werden dann entsprechend aufbereitete Daten an den Computer 6 weitergegeben. Für den Messpunkt 36 auf der Oberflächenlinie 21 des Körpers 22 würde die Messachse 4' des Messgerätes 1 eine Winkelabweichung zur Referenzachse 5 aufweisen, wenn sowohl das Messrad 15 wie auch das Führungsrad 16 ordnungsgemäss auf der Oberflächenlinie 21 aufliegen. In dieser Position würde der Sensor 7 einen Messwert von 0,96 und der Sensor 9 einen Messwert von 1,75 abgeben. Dieses Messwertpaar tritt nur beim Winkel von +30° auf, und die Position des Messgerätes 1, bzw. die Lage der Messachse 4, 4' ist deshalb genau bestimmbar. Dies gilt für jeden Punkt auf der Oberflächenlinie 21 sowohl bei stehendem wie auch bei gebeugtem oder liegendem Körper 22.

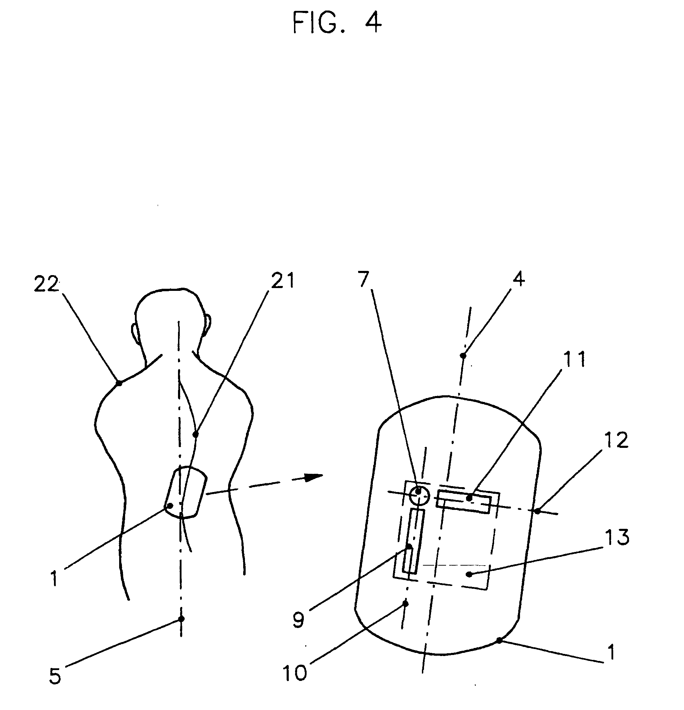

Die erfindungsgemässe Messanordnung ermöglicht gleichzeitig mit der Messung der Form und Länge der Oberflächenlinie 21 in der Sagittalebene, d.h. in einer Ebene parallel zur Bildebene der Fig. 2, die Erfassung der Form der Oberflächenlinie 21 in rechtwinklig dazu stehenden Frontalebenen. Wie in Fig. 4 dargestellt ist, ist dazu das Messgerät 1 mit einer zweiten Messeinrichtung 13 zur Ermittlung von Winkelabweichungen ausgestattet. Diese Messeinrichtung 13 umfasst den Beschleunigungssensor 9, welcher gleichzeitig zur ersten Winkelmesseinrichtung 3 gehört und zusätzlich einen dritten Beschleunigungssensor 11. Die Messachse 12 dieses dritten Beschleunigungssensors 11 steht senkrecht zur Messachse 10 des Beschleunigungssensors 9 und zur Messachse 8 des Beschleunigungssensors 7. Die beiden Messachsen 10 und 12 der beiden Beschleunigungssensoren 9 und 11 der Messeinrichtung 13 definieren eine Messebene, welche rechtwinklig zur Messebene der Messeinrichtung 3 verläuft. Diese Messebene, welche durch die beiden Messachsen 10 und 12 bestimmt ist, entspricht im dargestellten Beispiel der Bildebene der Fig. 4. Wird das Messgerät 1 entlang der Oberflächenlinie 21 verschoben, so erzeugt einerseits die Messeinrichtung 2 zur Bestimmung der Länge, bzw. von Streckensegmenten, entsprechende Messdaten und zu jeder Messposition werden von der Messeinrichtung 13 entsprechende Messdaten zur Bestimmung der Winkel abgegeben. Dabei werden, wie bereits zu Fig. 2 und 3 beschrieben, in entsprechender Weise die Messsignale der beiden Beschleunigungssensoren 9 und 11 zu Messwertpaaren zusammengefasst, welche eine eindeutige Bestimmung des Winkels der Messachse 4 des Messgerätes 1 zur Referenzachse, bzw. Gravitationsachse 5 in einer Frontalebene ermöglicht. Diese Messdaten werden wiederum mit der entsprechenden Software im Computer 6 verarbeitet, und es wird die Form der Oberflächenlinie 21, d.h. im beschriebenen Beispiel die Krümmung der Wirbelsäule in der Frontalebene (Skoliose) wiedergegeben.

Zur Messung des Verlaufs und der Form der Oberflächenlinie 21 in einer Sagittaloder Frontalebene genügen normalerweise die Messdaten, welche von der ersten oder zweiten Messeinrichtung 3, bzw. 13 zur Bestimmung von Winkelabweichungen ermittelt werden. Bei grossen Winkelabweichungen in beiden Ebenen kann es jedoch notwendig sein, die Auslenkung der Messebenen aus der idealen Vertikalebene zu beachten und zu kompensieren. Dies ist bei der erfindungsgemässen Anordnung der beiden Messeinrichtungen 3 und 13 möglich, da jeweils Messsignale eines dritten Beschleunigungssensors 11, bzw. 7 zur Verfügung stehen, welche Auslenkungen in einer zur Messebene rechtwinkligen Ebene anzeigen. Die von diesem dritten Messsensor 11, bzw. 7 erzeugten Messdaten werden zur Korrektur der Messwertpaardaten der beiden anderen Beschteunigungssensoren 7 und 9, bzw. 9 und 11 verwendet. Die entsprechende erfindungsgemässe Anordnung der drei Beschleunigungssensoren 7, 9, 11 kann in einfacher Weise in das Messgerät 1 eingebaut werden und ist kostengünstig und wenig störungsanfällig. Es ist auch möglich, Beschleunigungssensoren 7, 9, 11 mit verschiedenen Messkennlinien einzusetzen, wobei der Verlauf der Kennlinie in einem weiten Bereich zwischen linear und nicht linear ausgebildet sein kann. Die Messeinrichtung 2 zur Messung der Länge des Verschiebeweges entlang der Oberflächenkurve 21, wie auch die beiden Messeinrichtungen 3 und 13 zur Messung der Winkelveränderungen, können klein und kompakt gestaltet werden, so dass das Messgerät 1 sehr leicht und handlich ausgebildet werden kann. Insbesondere bei Einsatz einer drahtlosen Übermittlung der Daten zwischen dem Messgerät 1 und dem Computer 6 ergibt sich eine sehr gute Handlichkeit und Bedienbarkeit der Messanordnung. Durch die Anordnung von Steuerelementen in der Form des Eingabegerätes 32 mit den Bedienungstasten 19 und 20 sowie der Anzeigevorrichtung 41 am Messgerät 1 wird diese erleichterte Bedienbarkeit noch zusätzlich erhöht. Die entsprechenden Messungen lassen sich schneller und einfacher ausführen. Dies gilt nicht nur für die Vermessung der Form und Länge der Wirbelsäule, sondern auch für die Vermessung der Form und auch der Beweglichkeit von anderen Gelenken am menschlichen Körper 22 oder an anderen Objekten.

## Patentansprüche

1. Messanordnung zum Erfassen der Form und Länge einer Oberflächenlinie (21) eines Körpers (22) im Raum, mit einem frei beweglichen Messgerät (1), welches mit einer Messeinrichtung (2) zur Messung der Länge eines Verschiebeweges des Messgerätes (1) entlang der Oberflächenlinie (21) und einer Messeinrichtung (3) zur Bestimmung von Winkelveränderungen einer Messachse (4) des Messgerätes (1) zu einer vorgegebenen Referenzachse (5) ausgestattet ist, mit einer Datenübertragungseinrichtung zu einem Computer (6) und einem Computer (6), welcher die Weg- und Winkelmesswerte der Messeinrichtungen (2, 3) verarbeitet und eine Darstellung der Oberflächenlinie (21) erzeugt, wobei die Messeinrichtung (2) zur Messung der Länge des Verschiebeweges ein Messrad (15) und ein Führungsmittel (16) aufweist, welche mit Abstand zueinander angeordnet sind und zwei Auflagepunkte zur Oberflächenlinie (21) bilden, wobei diese beiden Auflagepunkte Punkte auf der Messachse (4) sind, **dadurch gekennzeichnet dass** die Messeinrichtung (3) zur Bestimmung von Winkelveränderungen zwei Beschleunigungssensoren (7, 9) mit je einer Messachse (8, 10) aufweist, die Messachsen (8, 10) dieser beiden Beschleunigungssensoren (7, 9) in einer gemeinsamen Ebene angeordnet sind und die beiden Messachsen (8, 10) in dieser Ebene rechtwinklig zueinander stehen und die Messachse (4) der Messeinrichtung (2) für den Verschiebeweg ebenfalls in dieser gemeinsamen Ebene angeordnet ist.

2. Messanordnung nach Patentanspruch 1, **dadurch gekennzeichnet, dass** zusätzlich zur ersten Messeinrichtung (3), mit den ersten und zweiten Beschleunigungssensoren (7, 9), zur Bestimmung von Winkelveränderungen eine zweite Messeinrichtung (13) zur Bestimmung von Winkelveränderungen vorhanden ist, diese zweite Winkelmesseinrichtung (13) einen dritten Beschleunigungssensor (11) und einen der beiden Beschleunigungssensoren (7, 9) der ersten Messeinrichtung (3) umfasst und die Messachse (12) dieses dritten Sensors (11) rechtwinklig zur Ebene der Messachsen (8, 10) der beiden Sensoren (7, 9) der ersten Messeinrichtung (3) steht.

3. Messanordnung nach Patentanspruch 1 oder 2, **dadurch gekennzeichnet, dass** die Referenzachse (5) zur Bestimmung der Winkelabweichungen die durch die Richtung der Erdbeschleunigung bestimmte Vertikalachse ist.

4. Messanordnung nach einem der Patentansprüche 1 bis 3, **dadurch gekennzeichnet, dass** jeder der Beschleunigungssensoren (7, 9, 11) mit einer Energiequelle (23) und mit einem Messwertaufnehmer (31) verbunden ist und der Messwertaufnehmer (31) über eine Schnittstelle (30) und eine Datenleitung (29; 27, 28) mit dem Computer (6) verbunden ist.

5. Messanordnung nach einem der Patentansprüche 1 bis 4, **dadurch gekennzeichnet, dass** die Messeinrichtung (2) für den Verschiebeweg mit einem Messwertaufnehmer (34) verbunden ist und dieser Messwertaufnehmer (34) über eine Schnittstelle (30) und eine Datenleitung (29; 27, 28) mit dem Computer (6) verbunden ist.

6. Messanordnung nach einem der Patentansprüche 1 bis 5, **dadurch gekennzeichnet, dass** das Messrad (15) der Messeinrichtung (2) für den Verschiebeweg mit einer Einrichtung zur Umformung der Längenwerte in digitale elektrische Signale ausgestattet ist.

7. Messanordnung nach einem der Patentansprüche 1 bis 6, **dadurch gekennzeichnet, dass** das frei bewegliche Messgerät (1) ein Eingabegerät (32) mit mindestens einer Steuertaste (19, 20), einen damit verbundenen Mikroprozessor (33) und eine Datenleitung (29; 27, 28) zum Computer (6) aufweist.

8. Messanordnung nach einem der Patentansprüche 1 bis 7, **dadurch gekennzeichnet, dass** die Verbindung zwischen dem frei beweglichen Messgerät (1) und dem Computer (6) aus einer Einrichtung zur drahtlosen Übertragung von Daten besteht, sowohl am frei beweglichen Messgerät (1) wie auch am Computer (6) je eine Sende-/Empfangseinheit (27, 28) angeordnet sind und alle Datenleitungen im Messgerät (1) mit dieser Sende-/Empfangseinheit (27) verbunden sind.

9. Messanordnung nach einem der Patentansprüche 1 bis 8, **dadurch gekennzeichnet, dass** das Messgerät (1) oder der Computer (6) mit einem Mikroprozessor (33) in der Form einer Ein-/Ausgabe-Einheit ausgestattet ist und dieser Mikroprozessor (33) als Schaltelement zwischen einem Eingabegerät (32) am Messgerät (1) und einem Eingabegerät (24) am Computer (6) ausgebildet ist

10. Messanordnung nach einem der Patentansprüche 1 bis 9, **dadurch gekennzeichnet, dass** das Messgerät (1) eine optische Anzeigevorrichtung (41) aufweist

11. Messanordnung nach Patentanspruch 10, **dadurch gekennzeichnet, dass** die optische Anzeigevorrichtung (41) aus mindestens einer Leuchtdiode oder einer Flüssigkristailanzeige besteht.

## Claims

1. A measuring arrangement for recording the shape and length of a surface line (21) of a body (22) in space, with a freely movable measuring instrument (1) which is equipped with a measuring device (2) for measuring the length of a displacement path of the measuring instrument (1) along the surface line (21) and with a measuring device (3) for determining angular variations of a measuring axis (4) of the measuring instrument (1) relative to a predetermined reference axis (5), with a data-transmission device linked to a computer (6) and with a computer (6) which processes the path-values and angle-values measured by the measuring devices (2, 3) and generates a representation of the surface line (21), the measuring device (2) for measuring the length of the displacement path exhibiting a measuring wheel (15) and a guide means (16), which are spaced from one another and which constitute two points of contact with the surface line (21), these two points of contact being points on the measuring axis (4), **characterised in that** the measuring device (3) for determining angular variations exhibits two acceleration sensors (7, 9) each with a measuring axis (8, 10), the measuring axes (8, 10) of these two acceleration sensors (7, 9) are arranged in a common plane and stand at right angles to one another in this plane, and the measuring axis (4) of the measuring device (2) for the displacement path is likewise arranged in this common plane.

2. Measuring arrangement according to Claim 1, **characterised in that** in addition to the first measuring device (3) for determining angular variations, with the first and second acceleration sensors (7, 9), a second measuring device (13) for determining angular variations is present, this second angle-measuring device (13) comprises a third acceleration sensor (11) and one of the two acceleration sensors (7, 9) of the first measuring device (3), and the measuring axis (12) of this third sensor (11) stands at right angles to the plane of the measuring axes (8, 10) of the two sensors (7, 9) of the first measuring device (3).

3. Measuring arrangement according to Claim 1 or 2, **characterised in that** the reference axis (5) for determining the angular deviations is the vertical axis defined by the direction of the acceleration due to gravity.

4. Measuring arrangement according to one of Claims 1 to 3, **characterised in that** each of the acceleration sensors (7, 9, 11) is connected to an energysource (23) and to a measured-value receiver (31) and the measured-value receiver (31) is connected to the computer (6) via an interface (30) and a data line (29; 27, 28).

5. Measuring arrangement according to one of Claims 1 to 4, **characterised in that** the measuring device (2) for the displacement path is connected to a measured-value receiver (34) and this measured-value receiver (34) is connected to the computer (6) via an interface (30) and a data line (29; 27, 28).

6. Measuring arrangement according to one of Claims 1 to 5, **characterised in that** the measuring wheel (15) of the measuring device (2) for the displacement path is equipped with a device for converting the length-values into digital electrical signals.

7. Measuring arrangement according to one of Claims 1 to 6, **characterised in that** the freely movable measuring instrument (1) exhibits an input device (32) with at least one control key (19, 20), a microprocessor (33) connected thereto and a data line (29; 27, 28) leading to the computer (6).

8. Measuring arrangement according to one of Claims 1 to 7, **characterised in that** the connection between the freely movable measuring instrument (1) and the computer (6) consists of a device for wireless transmission of data, a transmit/receive unit (27, 28) is arranged both on the freely movable measuring instrument (1) and on the computer (6), and all the data lines in the measuring instrument (1) are connected to said transmit/receive unit (27).

9. Measuring arrangement according to one of Claims 1 to 8, **characterised in that** the measuring instrument (1) or the computer (6) is equipped with a microprocessor (33) in the form of an input/output unit and this microprocessor (33) takes the form of a switching element between an input device (32) on the measuring instrument (1) and an input device (24) on the computer (6).

10. Measuring arrangement according to one of Claims 1 to 9, **characterised in that** the measuring instrument (1) exhibits an optical display device (41).

11. Measuring arrangement according to Claim 10, **characterised in that** the optical display device (41) consists of at least one light-emitting diode or of a liquid-crystal display.

## Revendications

1. Appareil de mesure en vue de la détection de la forme et de la longueur d'une ligne superficielle (21) d'un corps (22) dans l'espace, équipé d'un dispositif de mesure (1) librement mobile, qui est équipé d'un dispositif de mesure (2) en vue de la mesure de la longueur d'un déplacement du dispositif de mesure (1) le long de la ligne superficielle (21) et d'un dispositif de mesure (3) en vue de la détermination de variations angulaires d'un axe de mesure (4) du dispositif de mesure (1) par rapport à un axe de référence prédéterminé (5), d'un dispositif de transmission de données à un calculateur (6) et d'un calculateur (6) qui traite les valeurs de mesure de trajet et d'angle des dispositifs de mesure (2,3) et génère une représentation de la ligne superficielle (21), le dispositif de mesure (2) présentant en vue de la mesure de la longueur du trajet de déplacement une roue de mesure (15) et un moyen de guidage (16), qui sont disposés espacés l'un de l'autre et forment deux points d'appui par rapport à la ligne superficielle (21), ces deux points d'appui se trouvant sur l'axe de mesure (4), **caractérisé en ce que** le dispositif de mesure (3) présente, en vue de la détermination de variations angulaires, deux capteurs d'accélération (7,9) avec chacun un axe de mesure (8,10), les axes de mesure (8,10) de ces deux capteurs d'accélération (7,9) sont disposés dans un plan commun et les deux axes de mesure (8,10) se trouvent à angle droit l'un de l'autre dans ce plan et l'axe de mesure (4) du dispositif de mesure (2) pour le trajet de déplacement est disposé également dans ce plan commun.

2. Appareil de mesure selon la revendication 1, **caractérisé en ce qu'**en plus du premier dispositif de mesure (3), avec les premier et second capteurs d'accélération (7,9), en vue de la détermination de variations angulaires est prévu un second dispositif de mesure (13) en vue de la détermination de variations angulaires, ce second dispositif de mesure d'angle (13) comprend un troisième capteur d'accélération (11) et un des deux capteurs d'accélération (7,9) du premier dispositif de mesure (3) et l'axe de mesure (12) de ce troisième capteur (11) est disposé à angle droit par rapport au plan des axes de mesure (8,10) des deux capteurs (7,9) du premier dispositif de mesure (3).

3. Appareil de mesure selon la revendication 1 ou 2, **caractérisé en ce que** l'axe de référence (5) en vue de la détermination des écarts angulaires est l'axe vertical déterminé par la direction de l'accélération de la pesanteur.

4. Appareil de mesure selon une des revendications 1 à 3, **caractérisé en ce que** chacun des capteurs d'accélération (7,9,11) est relié à une source d'énergie (23) et à un enregistreur de valeurs de mesure (31) et l'enregistreur de valeurs de mesure (31) est relié par l'intermédiaire d'une interface (30) et d'une ligne de données (29; 27,28) au calculateur (6).

5. Appareil de mesure selon une des revendications 1 à 4, **caractérisé en ce que** le dispositif de mesure (2) pour le trajet de déplacement est relié à un enregistreur de valeurs de mesure (34) et cet enregistreur de valeurs de mesure (34) est relié par l'intermédiaire d'une interface (30) et d'une ligne de données (29; 27,28) au calculateur (6).

6. Appareil de mesure selon une des revendications 1 à 5, **caractérisé en ce que** la roue de mesure (15) du dispositif de mesure (2) pour le trajet de déplacement est équipé d'un dispositif de conversion des valeurs de longueur en signaux électriques numériques.

7. Appareil de mesure selon une des revendications 1 à 6, **caractérisé en ce que** le dispositif de mesure librement mobile (1) présente un dispositif d'entrée (32) équipé d'au moins une touche de commande (19,20), un microprocesseur (33) relié à celui-ci et une ligne de données (29;27,28) vers le calculateur (6).

8. Appareil de mesure selon une des revendications 1 à 7, **caractérisé en ce que** la liaison entre le dispositif de mesure librement mobile (1) et le calculateur (6) se compose d'un dispositif de transmission sans fil de données, non seulement sur le dispositif de mesure librement mobile (1), mais également sur le calculateur (6) sont disposées respectivement une unité d'émission/réception (27,28) et toutes les lignes de données dans le dispositif de mesure (1) sont reliées à cette unité d'émission/réception (27).

9. Appareil de mesure selon une des revendications 1 à 8, **caractérisé en ce que** le dispositif de mesure (1) ou le calculateur (6) est équipé d'un microprocesseur (33) sous la forme d'une unité d'entrée/sortie et ce microprocesseur (33) est réalisé sous la forme d'un élément de commutation entre un dispositif d'entrée (32) sur le dispositif de mesure (1) et d'un dispositif d'entrée (24) sur le calculateur (6).

10. Appareil de mesure selon une des revendications 1 à 9, **caractérisé en ce que** le dispositif de mesure (1) présente un dispositif d'affichage optique (41).

11. Appareil de mesure selon la revendication 10, **caractérisé en ce que** le dispositif d'affichage optique (41) se compose d'au moins une diode électroluminescente ou d'un affichage à cristaux liquides.
